(19) Europäisches Patentamt
European Patent Office
Office européen des brevets

(11) **EP 3 735 905 A1**

(12) **EUROPEAN PATENT APPLICATION**

(43) Date of publication:
**11.11.2020 Bulletin 2020/46**

(51) Int Cl.:
*A61B 6/00* (2006.01)   *G01N 23/041* (2018.01)

(21) Application number: **19173639.6**

(22) Date of filing: **09.05.2019**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Koninklijke Philips N.V.**
**5656 AG Eindhoven (NL)**

(72) Inventors:
• **YAROSHENKO, Andre**
 **5656 AE Eindhoven (NL)**
• **KOEHLER, Thomas**
 **5600 Eindhoven (NL)**

(74) Representative: **de Haan, Poul Erik et al**
**Philips International B.V.**
**Philips Intellectual Property & Standards**
**High Tech Campus 5**
**5656 AE Eindhoven (NL)**

(54) **APPARATUS FOR X-RAY DARK-FIELD AND/OR X-RAY PHASE CONTRAST IMAGING USING STEPPING AND MOIRÉ IMAGING**

(57)   Disclosed is an apparatus for X-ray dark-field and/or X-ray phase-contrast imaging. The apparatus has an imaging system, which includes a first and a second X-ray optical grating and an X-ray sensitive image detector having an ordered array of X-ray sensitive pixels. When no object is present, the first grating generates a fringe pattern in an entrance plane of the second grating. The second grating is configured to generate a Moire pattern from the fringe pattern so that the Moire pattern has a pitch which is less than 20 times a pixel pitch of the pixels of the X-ray sensitive image detector. The imaging system further comprises a controller, which is configured to control a relative movement between the second grating and the fringe pattern to acquire a Moiré-image at each of a plurality of different relative image acquisition positions when the object is present.

Fig. 1

EP 3 735 905 A1

**Description**

FIELD OF THE INVENTION

The present disclosure relates to an apparatus for X-ray dark-field and/or X-ray phase-contract imaging using phase stepping and Moire fringe analysis. The present invention also relates to a method of generating X-ray dark-field and/or X-ray phase-contrast images using this apparatus.

BACKGROUND OF THE INVENTION

In conventional techniques of X-ray imaging, an object is exposed to X-rays to obtain an attenuation image of the object. However, many object features do not have sufficient attenuation contrast so that they can be efficiently studied using attenuation-contrast imaging. In order to overcome this limitation, there have been major developments in recent years to obtain dark-field images and phase-contrast images using X-rays.

Some of these techniques can be classified as grating interferometric methods or grating non-interferometric methods. In each of these techniques, a fringe pattern of X-rays is generated after the X-rays have traversed the object. The changes in the fringe pattern induced by the object can be used to extract X-ray dark-field and X-ray phase-contrast images.

In order to determine the changes in the fringe pattern which are induced by the object, phase-stepping procedures or Moire fringe analysis have been applied. However, it has been shown that phase-stepping techniques typically require acquisition of ten or even more images in order to sufficiently suppress vibrational disturbances. This, however, leads to artefacts caused by patient motion. A long acquisition time also makes it difficult to apply this technique to X-ray computed tomography. Although, in principle, Moire fringe analysis offers the opportunity to obtain phase-contrast and dark-field information based on a single image, this technique results in a loss of spatial resolution compared to the phase stepping procedure.

Therefore, there is a need for an improved method and apparatus for generating X-ray phase-contrast and X-ray dark-field images.

SUMMARY OF THE INVENTION

According to an aspect of the present disclosure, an apparatus for X-ray dark-field and/or X-ray phase-contrast imaging is provided. The apparatus has an X-ray imaging system which comprises a first and a second X-ray optical grating and an X-ray sensitive image detector having an ordered array of X-ray sensitive pixels. The imaging system is configured to be usable with an X-ray beam which traverses the first grating, then the second grating before being incident on the X-ray sensitive detector for imaging an object, which is positioned in the beam path of the X-ray beam upstream of the first grating or between the first and the second grating. The imaging system is configured so that when no object is present, the first grating generates a fringe pattern in an entrance plane of the second grating. The second grating is configured to generate a Moire pattern from the fringe pattern so that the Moire pattern has a pitch which is less than 20 times or less than 10 times a pixel pitch of the pixels of the X-ray sensitive image detector. The imaging system further comprises a controller, which is configured to control a relative movement between the second grating and the fringe pattern to acquire a Moire-image at each of a plurality of different relative image acquisition positions.

The X-ray source may be a synchrotron or an X-ray source which generates X-rays using an electron beam which impinges on an anode. A diameter of a focal spot of the electron beam on the anode may be smaller than 2 mm or smaller than 1 mm. The diameter may be larger than 1 $\mu$m or larger than 10 $\mu$m.

The first grating may be configured as a phase grating and/or as an amplitude grating. Specifically, the first grating may be configured as a pure amplitude grating. The phase grating may be configured to cause a phase shift of $\pi$ or substantially $\pi$ between a portion of the X-rays which is transmitted through an aperture of the grating and a portion of the X-rays, which is transmitted through a partially opaque portion of the grating.

A pitch of the first grating may be less than 100 $\mu$m or less than 40 $\mu$m. The pitch of the first grating may be greater than 1 $\mu$m or greater than 5 $\mu$m.

A pitch of the second grating may be less than 200 $\mu$m or less than 80 $\mu$m. The pitch of the second grating may be greater than 2 $\mu$m or greater than 10 $\mu$m.

A pixel pitch of the detector may be smaller than 2 mm or smaller than 0.5 mm. The pixel pitch may be greater than 10 $\mu$m or greater than 50 $\mu$m.

The fringes of the fringe pattern may have a linear shape and/or maybe oriented parallel relative to each other.

The controller may be in signal communication with an actuator system of the X-ray imaging system which is configured to move a component of the X-ray imaging system based on signals received from the controller. By way of example, the actuator may include one or more piezo actuators.

The apparatus may include a data processing system for generating the dark-field and/or phase contrast image based on the acquired Moire images. The data processing system may include a processor and a memory for storing instructions processable by the processor. The processor may execute an operating system. The data processing system may further include an input and/or output unit configured to allow a user to receive data from the data processing system and/or to provide data to the data processing system. The computer system may further include a data storage system and/or a user interface for receiving user input.

The imaging system may be configured to generate the relative movement between the second grating and the fringe pattern by moving the first and/or the second grating. For each of the gratings, the movement may be within a plane of the respective grating. The imaging system may be configured so that the first and/or the second grating is controllably movable. The controlled movement of the first and/or second grating may be performed depending on control signals generated by the controller. The imaging system may include an actuator system which may include one or more actuators (such as a piezo actuator), which are in operative connection with the first and/or the second grating. The actuator system may be in signal communication with the controller. The actuator may actuate the relative movement between the first and the second grating depending on control signals received from the controller.

The system may be configured to at least partially generate the relative movement between the second grating and the fringe pattern by controllably displacing the first and/or the second grating.

The system may include an adjustment mechanism for adjusting a period of the Moire pattern. The adjustment mechanism may include an actuator. The actuator may be in signal communication with the controller. The actuator maybe in operative connection with the first and/or the second grating. The actuator maybe configured to adjust a relative position and/or a relative orientation between the first and the second grating based on signals received by the controller.

According to an embodiment, a pixel pitch of the pixels of the detector is smaller than 0.5 times or smaller than 0.3 times, or smaller than 0.2 times or smaller than 0.1 times a pitch of the Moire pattern.

According to a further embodiment, the system further comprises a data processing system, which is configured to generate an X-ray dark-field image and/or an X-ray phase-contrast image based on one or more analysis parameters. The system may be further configured to determine each of the one or more analysis parameters based on a plurality of pixel data values of one of the Moire images, which sample a Moire fringe pattern of the Moire image over a period of the Moire fringe pattern.

The one or more analysis parameters may further be generated based on a position parameter of the image acquisition positions. The position parameter may be defined to be measured in a direction perpendicular to an axis of the second grating and within the entrance plane of the second grating. The data processing system may further be configured to calculate the dark-field image and/or the phase contrast image based on the determined analysis parameter.

According to a further embodiment, the data processing system is further configured to determine each of the one or more analysis parameters further based on pixel data values of pixels of different ones of the Moire images. The Moire images may be acquired at different relative image acquisition positions. The pixels taken from the different Moire images may relate to a same position (e.g. a same detector pixel) on an active surface of the image detector.

According to a further embodiment, the imaging system is configured so that the Moire pattern is at least partially caused by an axis of fringes of the fringe pattern being rotated relative to an axis of the second grating as seen in the entrance plane of the second grating. Additionally or alternatively, the Moire pattern may be at least partially caused by a difference between a grating pitch of the second grating and a pitch of the fringe pattern.

The Moire pattern may be at least partially caused by a deviation of a relative position and/or a relative orientation between the first and the second grating from an ideal configuration of a Talbot interferometer or a Talbot-Lau interferometer. In Talbot interferometers and Talbot-Lau interferometers, the first and the second grating are separated by a fractional or integer Talbot distance and the grid axes of the first and the second grating are oriented parallel relative to each other.

According to a further embodiment, the X-ray imaging system is configured so that the first and the second grating form a grating interferometer. The apparatus maybe configured so that the first grating acts as a diffraction grating. The diffraction grating may generate a diffraction pattern, which fills the space between the first and the second grating. The grating interferometer may be configured as a Talbot interferometer or as a Talbot-Lau interferometer. The fringe pattern may represent a cross-section through a spatial Talbot pattern (also denoted as Talbot carpet). The Talbot pattern may fill the space between the first and the second grating. A distance between the first grating and the second grating may be or may substantially be a fractional or integer Talbot distance. Through a distance, which deviates from an exact fractional or integer Talbot distance (and thereby only substantially represents a fractional or integer Talbot distance), it is possible to generate a fringe pattern in the entrance plane of the second grid, which has a pitch, which deviates from the grating pitch of the second grid. This allows generation of a Moire pattern from the fringe pattern using the second grid.

Alternatively, the imaging system may be configured for X-ray edge illumination. The first grating may form a plurality of beamlets for X-ray edge illumination of the second grating. The imaging system may be configured so that downstream of the first grating, a plurality of separate beamlets are formed. The beamlets may be configured to be separate from each other. Therefore, the beamlets do not fill the space between the first and the second grating. The first and second grating, which are configured for edge illumination, may be further configured so that the fringe pattern in the entrance plane of the second grating represents a projection image of the apertures of the first grating. The projection image may be an unmagnified or magnified projection image.

According to a further embodiment, the system further comprises a third grating, which is arranged in the beam path of the X-ray beam upstream of the first grating. The imaging system may be configured to at least partially generate the relative movement between the second grating and the fringe pattern by controllably displacing the first, the second and/or the third grating. The apparatus may include an actuator system which includes one or more actuators. The actuators may include piezo actuators. The actuators may be in operative connection with the first, second and/or third grating. The actuator may be configured to displace a grating, which is in operative connection with the actuator, within a plane of the grating and in a direction perpendicular to a grating axis of the grating. The actuator system may be in signal communication with the controller. The actuator may displace the first, second and/or third grating in response to control signals received from the controller.

The third grating may be configured as a pure amplitude grating. A distance between the third and the first grating maybe less than 2 m or less than 1 m. The distance may be greater than 2 cm or greater than 10 cm. A pitch of the third grating may be less than 200 $\mu$m or less than 20 $\mu$m. The pitch of the third grating may be greater than 1 $\mu$m or greater than 3 $\mu$m. A slit width of the third grating may be less than 60% or less than 50% of the pitch. The slit width may be greater than 5% or greater than 20% of the pitch.

According to a further embodiment, the X-ray imaging system is configured to determine, based on the Moire images: (a) a parameter of a spatial position of the fringe pattern in the entrance plane, measured in a direction perpendicular to a fringe axis of the fringe pattern; and/or (b) a parameter of an amplitude of the fringe pattern. The parameter of the spatial position and/or the parameter of the amplitude may be local parameters, which represent local properties of the fringe pattern in the entrance plane of the second grating. Additionally or alternatively, the parameters may represent a change of a fringe pattern acquired from an object relative to a fringe pattern acquired when no object is present. Additionally, the X-ray imaging system may further be configured to determine, depending on the Moire images, a parameter of an average pixel data value of the fringe pattern representing an average across at least a period of the fringe pattern. The parameter of the average may represent a local parameter, which represents a local property of the fringe pattern in the entrance plane of the second grating. Additionally or alternatively, the parameter of the average may represent a change of a fringe pattern acquired from an object relative to a fringe pattern acquired when no object is present.

According to a further aspect of the present disclosure, a method for generating X-ray dark-field and/or X-ray phase-contrast images using an apparatus having an X-ray imaging system is provided. The imaging system comprises an X-ray sensitive image detector having an ordered array of X-ray sensitive pixels, a first and a second X-ray optical grating. The imaging system is configured to be usable with an X-ray beam which traverses the first grating, then the second grating before being incident on the detector for imaging an object, which is positioned in the beam path of the X-ray beam upstream of the first grating or between the first and the second grating. The imaging system is further configured so that when no object is present, the first grating generates a fringe pattern in an entrance plane of the second grating. The method comprises generating a Moire pattern from the fringe pattern using the second grating so that the Moire pattern has a pitch which is less than 20 pixels or less than 10 pixels of the detector. The method further comprises controlling, using a controller, a relative movement between the second grating and the fringe pattern to acquire a Moire-image at each of a plurality of different relative image acquisition positions.

According to a further aspect of the present disclosure, a method for generating an object image based on at least two fringe pattern images is provided. Each of the fringe pattern images shows a different fringe pattern. In each of the fringe pattern images, the fringe pattern has a pitch which is less than 20 pixels or less than 10 pixels of the respective image and a pixel pitch of the pixels of the respective image is smaller than 0.5 times or smaller than 0.3 times, or smaller than 0.2 times or smaller than 0.1 times the pitch of the fringe pattern of the respective image. The method comprises reading and/or generating, using a data processing system, the fringe pattern images and one or more position parameters for each of the fringe pattern images. The method further comprises determining, using the data processing system, one or more analysis parameters, wherein each of the one or more analysis parameters is determined based on the position parameters and further based on a plurality of pixel data values of a plurality of pixels of the images which include: a) pixels of one of the images, which sample the fringe pattern of the respective image over a period of the fringe pattern; and b) pixels of different ones of the images. The method further comprises determining at least one pixel of the object image based on the one or more analysis parameters.

Each of the pixels, which are taken from different ones of the fringe pattern images, may have a same or substantially a same pixel position within the respective image. The object image may show an object to be imaged. The object image may represent a phase-contrast image or a dark-field image.

Each of the position parameters may represent a parameter of a relative image acquisition position. The relative image acquisition position may relate to a relative position between a grid and a fringe pattern. The grid and fringe pattern may be generated using a Talbot interferometer, a Talbot-Lau interferometer, and/or a system for edge illumination phase-contrast and/or dark-field imaging.

According to a further embodiment, the one or more analysis parameters are parameters of a model function. The model function may represent a fringe pattern, in particular a linear fringe pattern. The determining of the one or more analysis parameters may comprise fitting the model function to the plurality of pixel data values using the position parameters. The model function may include a trigonometric function, such as a cosine and/or a sinus function.

According to a further embodiment, the one or more analysis parameters comprise a parameter of a spatial position, in particular a parameter of a phase or a phase shift of the model function. Additionally or alternatively, the one or more analysis parameters may comprise a parameter of an amplitude of the model function. The spatial position may be a position within the image plane. The spatial position may be a spatial position in a direction perpendicular or substantially perpendicular to a fringe axis of a fringe pattern represented by the model function. The amplitude may be an amplitude of a periodic function such as the amplitude of a cosine function or the amplitude of a sine function. The amplitude may be a level of a local extremum relative to an average level of the model function. If the model function represents a periodic function, the average level may be determined by averaging values of the model function over one or more periods of the model function.

According to a further embodiment, the method further comprises determining, for each of a plurality of pixels of the object image, one or more analysis parameters. Each of the analysis parameters may be determined based on the position parameters and further based on a plurality of pixel data values of a plurality of pixels of the fringe pattern images and selected for the respective object image pixel. The pixels of the fringe pattern images may include: a) pixels of one of the fringe pattern images, which sample the fringe pattern of the respective fringe pattern image over a period of the fringe pattern. The fringe pattern image may be selected for the respective object pixel or may be the same for each of the object pixels. The pixels of the fringe pattern images for the respective object image pixel may further include b) pixels of different ones of the fringe pattern images.

For each of the object image pixels, the set of pixels of the fringe pattern images which are used for determining the object image pixel may be different. Specifically, the sets may be overlapping or non-overlapping.

According to a further aspect of the present disclosure, a program element for generating an object image based on at least two fringe pattern images is provided. Each of the fringe pattern images shows a different fringe pattern. In each of the fringe pattern images, the fringe pattern has a pitch which is less than 20 pixels or less than 10 pixels of the respective image and a pixel pitch of the pixels of the respective image is smaller than 0.5 times, or smaller than 0.3 times, or smaller than 0.2 times or smaller than 0.1 times the pitch of the fringe pattern of the respective image. The object image is generated using a data processing system, wherein the program element, when being executed by a processor of the data processing system, is adapted to carry out: reading and/or generating, using the data processing system, the fringe pattern images and one or more position parameters for each of the fringe pattern images; determining, using the data processing system, one or more analysis parameters, wherein each of the one or more analysis parameters is determined based on the position parameters and further based on a plurality of pixel data values of a plurality of pixels of the images, which include: a) pixels of one of the images, which sample the fringe pattern of the respective image over a period of the fringe pattern; and b) pixels of different ones of the images. The program element, when being executed by a processor of the data processing system, is further adapted to carry out determining at least one pixel of the object image based on the one or more analysis parameters.

According to a further aspect, a computer readable medium is provided having stored thereon the computer program element described in the preceding paragraph.

BRIEF DESCRIPTION OF THE DRAWINGS

Fig. 1 is as schematic perspective illustration of an apparatus for X-ray dark-field and X-ray phase-contrast imaging according to a first exemplary embodiment;

Fig. 2 is a schematic illustration of the imaging process in the apparatus according to the first exemplary embodiment, which is shown in Fig. 1;

Figs. 3A to 3C are schematic illustrations of the change in the average signal, the change in the amplitude and the change in the phase of the fringe pattern measured using the apparatus according to the first exemplary embodiment which is shown in Fig. 1;

Fig. 4 is a Moire image acquired using the apparatus according to the first exemplary embodiment, which is shown in Fig. 1;

Fig. 5 is a schematic illustration of an apparatus for X-ray phase-contrast imaging and X-ray dark-field imaging according to a second exemplary embodiment;

Fig. 6 is a flowchart of an exemplary method for analyzing the images which have been acquired using the apparatus according to the first exemplary embodiment, which is shown in Fig. 1 or the apparatus according to the second exemplary embodiment, which is shown in Fig. 5.

## DETAILED DESCRIPTION OF EMBODIMENTS

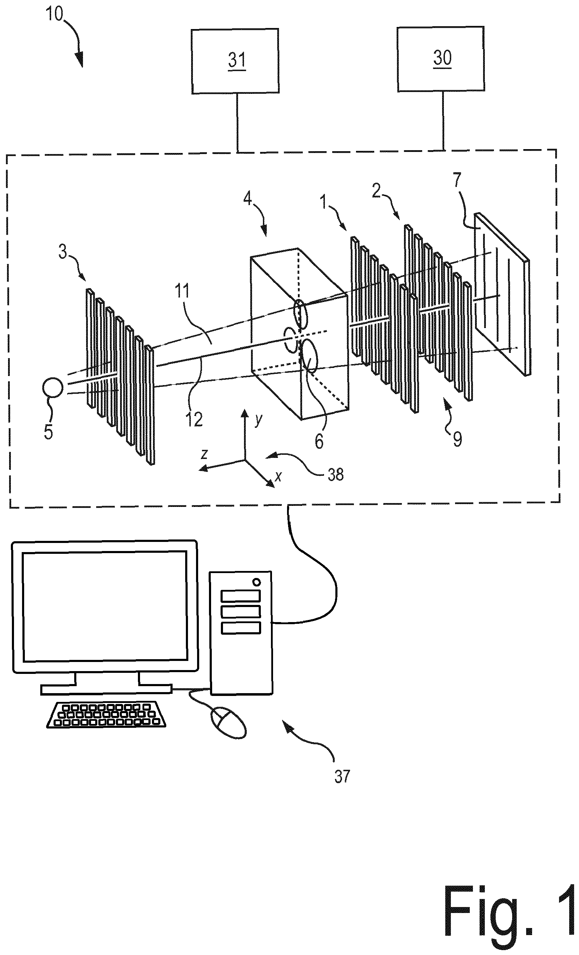

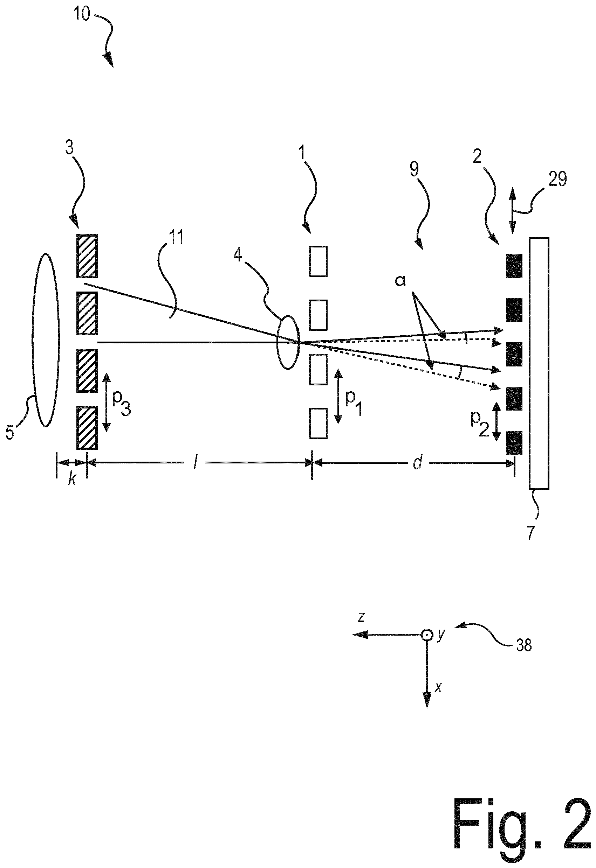

Fig. 1 is a schematic perspective illustration of an apparatus 10 according to a first exemplary embodiment, which is configured for X-ray dark-field imaging and X-ray phase-contrast imaging. Fig. 2 schematically illustrates the imaging process in the apparatus 10. The apparatus 10 has a grating interferometer, which includes a first grating 1, a second grating 2 and a third grating 3, which are arranged in a beam path of a divergent X-ray beam 11. The second grating 2 is arranged in the beam path downstream of the first grating 1. The first grating 1 and the second grating 2 are arranged so that their grating planes are parallel relative to each other or substantially parallel relative to each other. The first grating may be a pure amplitude grating or a phase grating, which shifts the phase of the portion of the X-ray beam 11, which traverses a semi-transparent portion of the phase grating. By way of example, the phase shift amounts to $\pi$ or $\pi/2$.

In the illustrated embodiment, the object 4 under inspection is disposed in the beam path of the X-ray beam 11 between the third grating 3 and the first grating 1. However, it is also conceivable that the object 4 is in the beam path of the X-ray beam 11 between the first grating 1 and the second grating 2.

The X-ray beam 11 is generated using an X-ray source 5 of the apparatus 10. The X-ray source 5 may be configured as an X-ray tube, which generates a focus of an electron beam on a surface of an anode. The anode may be configured as a rotating anode disk. Alternatively, the X-ray source may be configured as a synchrotron. If a synchrotron is used as the X-ray source 5 or if the source size of an X-ray tube is small enough (such as smaller than 1 square millimeter), it is possible to operate the apparatus 10 without a third grating 3. The third grating 3 is a pure amplitude grating and provides a plurality of apertures, each of which creating a sufficiently coherent virtual line source.

In the exemplary embodiment, the third grating 3 is placed close to the source 5. By way of example, a distance $k$ (shown in Fig. 2) of the third grating 3 from the source may be smaller than 40 cm or smaller than 5 cm.

As it is illustrated in Fig. 2, it has been shown that improved dark-field and phase-contrast images can be obtained if the following equation is satisfied:

$$p_3 = \frac{p_2 l}{d},$$

with $p_3$ being the pitch (i.e. period) of the third grating 3, $p_2$ being the pitch of the second grating 2, $l$ being the distance between the third grating 3 and the first grating 1 and $d$ being the distance between the first grating 1 and the second grating 2. The above equation ensures that each line source generated using the third grating 3 contributes constructively to the image-formation process.

The illumination of the first grating 1 using the X-ray beam 11, which is generated using the source 5, and which traverses the third grating 3, generates a spatial Talbot pattern (also denoted as Talbot carpet), which fills the space 9 between the first grating 1 and the second grating 2. The first grating 1 therefore acts as a diffraction grating. The distance between the first grating 1 and the second grating 2 corresponds or substantially corresponds to a fractional Talbot distance or to an integer Talbot distance. Thereby, in the entrance plane of the second grating 2, a fringe pattern is generated formed by linear fringes.

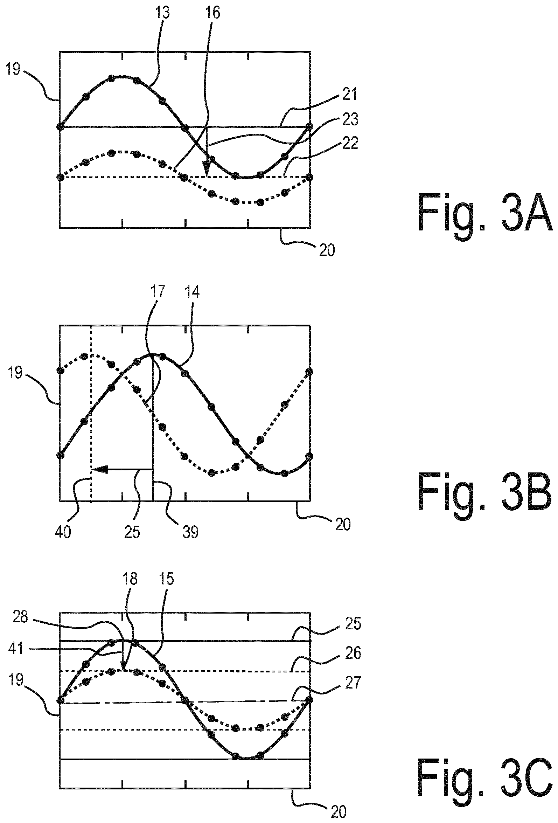

It has been shown that a comparison between a first fringe pattern which is generated when the X-ray beam 11 is transmitted through the object 4 and a second fringe pattern, which is generated when no object is present, allows generation of X-ray dark-field and X-ray phase-contrast images of the object 4. This is explained in more detail in relation to Figs. 2 and 3A to 3C.

Specifically, as is illustrated in Fig. 2, a phase object 4, which is placed in the beam path of the X-ray beam 11 causes the X-ray beam 11, which is transmitted through the object, to be slightly refracted, thereby generating a phase shift in the wavefront of the X-ray beam, which causes the fringes of the fringe pattern in the entrance plane of the second grating 2 to be displaced by an angle $\alpha$. The angle $\alpha$ is directly proportional to a local directional derivative of the wavefront phase profile $\Phi$. The directional derivative is also denoted as the differential phase contrast and expressed by the equation:

$$\alpha = \frac{\lambda d}{p_2} \frac{\partial \Phi(x, y)}{\partial x},$$

where $x$ and $y$ are the cartesian coordinates in the entrance plane of the second grating (see the coordinate systems 38 shown in Figs. 1 and 2) and $d$ (shown in Fig. 2) denotes the distance between the first grating 1 (close to which the object is placed in this exemplary embodiment) and the second grating 2. In configurations in which the object 4 is placed between the first grating 1 and the second grating 2, *d* should be replaced by the distance between the object 4 and the third grating 3. The *x*-axis is oriented perpendicular to the axis of the fringes. Therefore, by measuring, for each pixel of the detector 7, a local displacement of the fringe pattern in the entrance plane of the second grating 2, it is possible to generate a differential phase-contrast image. Based on this image, a phase-contrast image can be obtained by integration of the pixel data values which represent the differential phase contrast.

Further, specimens, which produce small-angle X-ray scattering contributions show a decrease of the amplitude of the fringe pattern. Therefore, by measuring, for each pixel of the detector 7, the change in the amplitude of the fringe pattern which is caused by the object (i.e. by measuring the amplitude with and without the object), it is possible to generate a dark-field image.

Figs. 3A to 3C are plots of the intensity of the fringe pattern (y-axis), as measured in the entrance plane of the second grating versus position (x-axis). The position is measured along an axis in the entrance plane, which is oriented perpendicular to the axis of the fringes.

The dotted curves 16, 17 and 18 in Figs. 3A to 3C indicate the intensity which is measured when the X-rays have traversed an object, whereas the solid curves 13, 14 and 15 in Figs. 3A to 3C indicate the intensity, which is measured when no object is present.

For the sake of simplicity, the dotted curve in Fig. 3A illustrates the intensity of an object model, which attenuates the X-ray beam without causing a differential phase shift and without causing small-angle scattering. The dotted curve of Fig. 3B illustrates the intensity of an object model, which causes a differential phase shift without attenuating the X-ray beam and without causing small-angle scattering. Fig. 3C illustrates the intensity of an object model, which causes small-angle scattering without causing a differential phase shift and without attenuating the X-ray beam.

As can be seen from Fig. 3A, the attenuation of the X-ray beam leads to a decrease of the average intensity from a first level 21 to a second level 22, as is indicated by arrow 23. The average is calculated over a period of the fringe pattern. As can be seen from Fig. 3B, the differential phase shift generated by the object causes a spatial shift of the fringe pattern in a direction perpendicular to the fringe axis of the fringe pattern from a position 39 to a position 40, as is indicated by arrow 25. Further, as can be seen from Fig. 3C, the small angle scattering by the object reduces the amplitude of the intensity of the fringe pattern from a level 25 to a level 26, as is indicated by arrow 41. The amplitude is measured as an intensity level at a local extremum (such as the extremum 28) relative to an average intensity level 27, which represents an average over one period of the fringe pattern.

Returning back to Fig. 1, since the pixel pitch of the array of X-ray sensitive pixels of the detector 7 is greater than the pitch of the fringe pattern in the entrance plane of the second grating 2, the apparatus 10 is configured to perform an electronically controllable relative movement between the second grating and the fringe pattern to sample the fringe pattern at a plurality of different relative image acquisition positions. In the exemplary embodiment, the pixel pitch of the detector 7 may be greater than 10 $\mu$m or greater than 50 $\mu$m. The pixel pitch may be smaller than 2 mm or smaller than 0.5 mm.

The direction vector of the relative movement is angled (such as perpendicular of substantially perpendicular) relative to the fringe axis of the fringe pattern and/or the grating axis of the second grating 2.

The relative movement is controlled by a controller 30 of the imaging system, which is in signal communication with an actuator (not shown in Fig. 1) of the imaging system. The actuator may be configured as a piezo actuator. The actuator is configured to actuate the relative movement between the fringe pattern and the second grating 2 based on control signals received from the controller 30.

The circular dots on the curves of Figs. 3A to 3C indicate the fringe pattern intensity which has been recorded using the relative movement between the second grating and the fringe pattern. Based on the fringe pattern images which have been acquired, it is possible to determine, for each of the detector pixels of the detector 7 (shown in Fig. 1), a parameter $D_i$ of the local amplitude of the fringe pattern (*i* being an index designating the pixel), a parameter $\psi_i$ of the local phase of the fringe pattern and a parameter $T_i$ of a local intensity of the fringe pattern.

The apparatus of the first exemplary embodiment may be configurable so that the grating pitch of the second grating is equal to or substantially equal to the pitch of the fringe pattern in the entrance plane of the second grating. In this configuration, the parameters $D_i$, $\psi_i$ and $T_i$ can be obtained using the intensity values $f_{ij}$ measured using the *i*-th pixel (*j* being an index designating the fringe pattern image). Specifically, the parameters $D_i$, $\psi_i$ and $T_i$ can be obtained by varying these parameters to minimize $\Delta_i^2(T_i,\ D_i, \psi_i)$ which is defined by the following equation:

$$\Delta_i^2(T_i,\ D_i, \psi_i) = \sum_j \frac{1}{\mathrm{Var}(f_{ij})}\left(f_{ij} - T_i A_i\left(1 + D_i V_i \cos\left(\psi_i + \phi_i + 2\pi x_j\right)\right)\right)^2,$$

wherein $x_j$ denotes the fraction of the period of the fringe pattern which corresponds to the image acquisition position of the $j$-th fringe pattern image. The parameters $A_i$, $V_i$, and $\phi_i$ for each pixel $i$ in the above equation can be obtained by acquiring a plurality of fringe pattern images when no object is present. $\text{Var}(f_{ij})$ denotes a variance estimate for the measurement $f_{ij}$. A simple estimate for the variance is $\text{Var}(f_{ij}) = f_{ij}$. Since a pixel of the detector receives X-ray radiation transmitted through a plurality of slits of the second grating, the parameters $A_i$, $V_i$, and $\phi_i$ for the i-th pixel represent the local behavior of the fringe pattern averaged over the area of the i-th pixel.

The minimum number of measurements which are necessary for acquiring intensity samples based on the above equation so that the phase shift and the change of the amplitude can be determined is three. However, it has been shown by the inventors that due to mechanical vibrations, it is typically necessary to acquire 10 or even more images to ensure that a sufficient image quality is obtained. This in turn, however, may cause image artefacts due to patient motion (such as respiratory movements or heart beat) so that the resulting images do not qualify as diagnostic images.

However, the inventors have found that it is possible to overcome these problems. Specifically, the inventors have shown that X-ray dark-field imaging as well as X-ray phase-contrast imaging is possible with a comparatively low number of images if the second grating is configured to generate a Moire pattern from the fringe pattern in the entrance plane of the second grating. The Moire pattern is generated so that it has a period which is less than 20 times or less than 10 times a pixel pitch of the X-ray sensitive image detector. In other words, a Moire pattern is generated at an exit plane of the second grating (shown in Fig. 1) from the fringe pattern, which is generated in the entry plane of the second grating 2.

By way of example, the imaging system may be configured so that the Moire pattern is caused by one or a combination of a deviation of a relative position and/or a relative orientation between the first and second gratings from an ideal Talbot configuration. By way of example, the distance between the first grating and the second grating may be selected so that the pitch of the fringe pattern in the entrance plane of the second grating, is different from the pitch of the second grating. Additionally or alternatively, the grating plane of the first grating may be inclined relative to the grating plane of the second grating. The inclination may be caused by a rotation of an axis of the first grating relative to an axis of the second grating. Additionally or alternatively, the grating axis of the first grating may be rotated relative to the grating axis of the second grating about an axis which is perpendicular to the grating plane of the first and/or second grating. These configurations allow switching between Moire pattern imaging and non-Moire pattern imaging by providing an actuator, which is configured for actuating a relative movement between the first and the second grating. Additionally or alternatively, it is also conceivable that the grating pitch $p_1$ (shown in Fig. 2) of the first grating 1 and/or the grating pitch $p_2$ of the second grating 2 are adapted to generate Moire images.

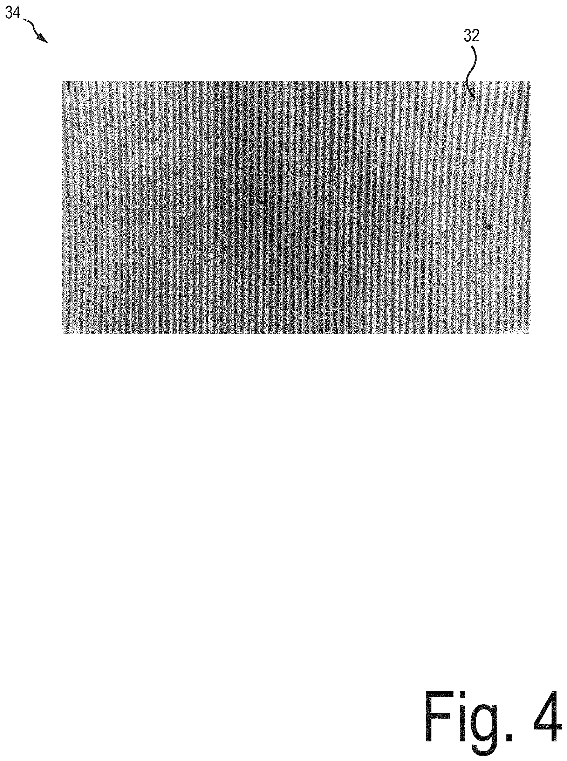

Fig. 4 shows an example of a Moire image 34 acquired using the image detector 7 (shown in Fig. 1). In the Moire image 35 of Fig. 4, the fringe pattern has fringes 32 which are not exactly linear (but rather substantially linear) and which are not exactly parallel (but rather substantially parallel) relative to other fringes of the Moire image 34. This is caused by a deviation of the gratings from a perfect plane.

The Moire pattern therefore represents an irregular sampling of the fringe pattern in the entrance plane of the second grating 2 (shown in Fig. 1). The irregular sampling allows using a plurality of neighboring or substantially neighboring pixel data values acquired using the detector 7 (shown in Fig. 1) to determine a parameter of a local phase shift of the fringe pattern, a parameter of a local average intensity of the fringe pattern and a parameter of a local average intensity of the fringe pattern. The spatial resolution achieved with this technique is limited by the spatial extent of the neighboring or substantially neighboring pixels, which are used to determine the parameters. Since the Moire pattern has a period which is less than 20 times or less than 10 times the pixel pitch of the X-ray sensitive image detector, the neighboring or substantially neighboring pixels can be selected so that their spatial extent is comparatively small.

Further, in the exemplary embodiment, the pixel pitch of the pixels of the detector is smaller than 0.5 times or smaller than 0.3 times or smaller than 0.2 times or smaller than 0.1 times the period of the Moire pattern. This allows resolving the Moire pattern with a sufficient accuracy.

Specifically, based on the plurality of Moire images acquired at different image acquisition positions provided by the relative movement between the second grating and the fringe pattern at the entrance plane of the second grating, for each of a plurality of pixels, a parameter $D_i$ of the local amplitude of the fringe pattern ($i$ being an index designating the pixel), a parameter $\psi_i$ of a local phase shift of the fringe pattern and a parameter $T_i$ of a local average intensity of the fringe pattern can be determined. These analysis parameters $D_i$, $\psi_i$ and $T_i$ can be obtained by varying these parameters to minimize $\Delta_i^2(T_i, D_i, \psi_i)$ in the following equation:

$$\Delta_i^2(T_i, D_i, \psi_i) = \sum_{j, i' \in \mathcal{N}_i} \frac{1}{\mathrm{Var}(f_{i'j})} \left( f_{i'j} - T_i A_{i'} \left( 1 + D_i V_{i'} \cos\left( \psi_i + \phi_{i'} + 2\pi x_j \right) \right) \right)^2,$$

wherein $\mathcal{N}_i$ is a set of pixel indices, which is formed by pixels in the neighborhood of pixel $i$. The $i$-th pixel may, but not necessarily, be an element of $\mathcal{N}_i$. The set of pixels $\mathcal{N}_i$ may be chosen so that the pixel data values of the set of pixels sample an intensity fluctuation within a same period of the Moire-pattern.

Accordingly, since the Moire pattern allows using a plurality of pixel data values of different pixels from a same Moire image to sample the fringe pattern at different phase positions, it is possible to determine the parameters $D_i$, $\psi_i$ and $T_i$ with a sufficient accuracy using a small number of Moire images, such as only two Moire images. Since for each pixel $i$ on the detector, the parameters $D_i$, $\psi_i$ and $T_i$ are chosen based on the pixel set $\mathcal{N}_i$, the resolution of the images, which can be obtained using these parameters is limited by the spatial extent of the pixel set $\mathcal{N}_i$. However, since the proposed method uses a plurality of Moire images which are acquired at different relative image acquisition positions between the second grating and the fringe pattern in its entrance plane, a sufficiently high image quality can be obtained, even if the number of pixels $\mathcal{N}_i$ is comparatively small and represent a small spatial extent.

In the foregoing equation, the parameters $A_i$, $V_i$, and $\phi_i$ for each pixel $i$ can be obtained by acquiring a plurality of fringe pattern images when no object is present. $\mathrm{Var}(f_{ij})$ denotes a variance estimate for the measurement $f_{ij}$.

Therefore, using the proposed system and method, it is possible to generate dark-field images and phase-contrast images at a high spatial resolution based on only a small number of images acquired from the object.

The inventors have further shown that even better values for the analysis parameters $D_i$ and $T_i$ can be obtained if a second minimization step is performed which minimizes $\Delta_i^2(T_i, D_i)$ in the equation:

$$\Delta_i^2(T_i, D_i) = \sum_j \frac{1}{\mathrm{Var}(f_{ij})} \left( f_{ij} - T_i A_i \left( 1 + D_i V_i \cos\left( \psi_i + \phi_i + 2\pi x_j \right) \right) \right)^2,$$

wherein the parameters $D_i$ and $T_i$ are varied to minimize $\Delta_i^2(T_i, D_i)$ and $\psi_i$ is kept at the value retrieved from the first optimization step. It has been shown that the second minimization step according to the above equation leads to a higher accuracy for the analysis parameters $D_i$ and $T_i$, especially in cases in which the differential phase varies more slowly in space than the transmission and dark-field.

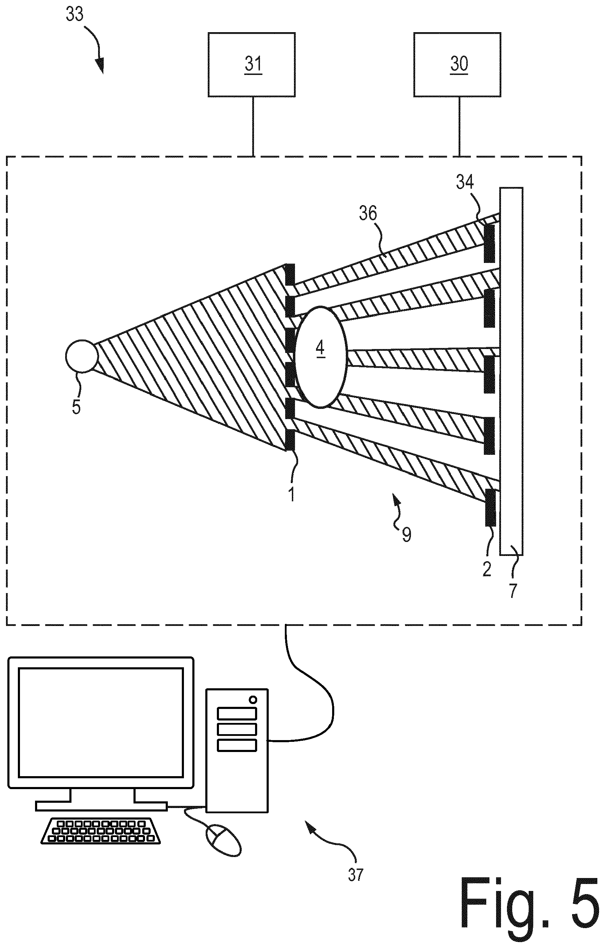

Fig. 5 schematically shows an apparatus 33 for generating X-ray phase-contrast images and X-ray dark-field images according to a second exemplary embodiment. In the second embodiment, the apparatus 33 is configured for edge illumination phase-contrast and dark-field imaging. Edge illumination is a non-interferometric and incoherent X-ray imaging technique. Like in the first exemplary embodiment described above, the images acquired using edge illumination contain a mixture of attenuation and refraction (or differential phase) contrast, the latter being proportional to the spatial derivative of the X-ray phase shift.

The apparatus 33 comprises a first grating 1 and a second grating 2, each of which being configured as a pure absorption mask. The first grating 1 shapes the incident radiation into an array of beamlets (such as the beamlet 36) so that the openings in the first grating 1 form a plurality of incoherent X-ray line sources. Each of the beamlets traverses the object 4 and is incident on an entry plane of the second grating 2. The beamlets form a fringe pattern in the entrance plane of the second grating 2. Therefore, unlike the apparatus 10 of the first exemplary embodiment (shown in Fig. 1), the first grating 1 of the apparatus 33 of the second exemplary embodiment does not function as a diffraction grating which generates a spatial interference pattern which fills the space 9 between the first grating 1 and the second grating 2.

The apparatus 33 according to the second exemplary embodiment is configured for a controllable relative movement between the second grating 2 and the fringe pattern which is generated at the entrance plane of the second grating 2 for acquiring, using the image detector 7, an image at each of a plurality of relative image acquisition positions. The relative movement may be actuated by an actuator (not shown in Fig. 5) which is in operative connection with the first and/or the second grating for moving the first and/or second grating in a direction which has a component perpendicular to the grating axis of the movable respective grating. The actuator maybe in signal communication with a controller 30 of the apparatus 33 and actuates the relative movement between the fringe pattern and the second grating 2 in response to a control signal received from the controller 30.

Further, the apparatus 33 is also configured so that the second grating 2 generates a Moire pattern from the fringe pattern which is generated in the entrance plane of the second grating 2. The Moire pattern may be generated by configuring the first grating 1, the second grating 2 and a position and/or orientation between the first grating 1 and second grating 2 in a manner as has been described in connection with the first exemplary embodiment.

Thereby, the apparatus 33 is configured to acquire a Moire image at each of a plurality of image acquisition positions provided by the relative movement between the fringe pattern and the second grating. The analysis parameters can then be determined in the same way as explained in connection with the first exemplary embodiment.

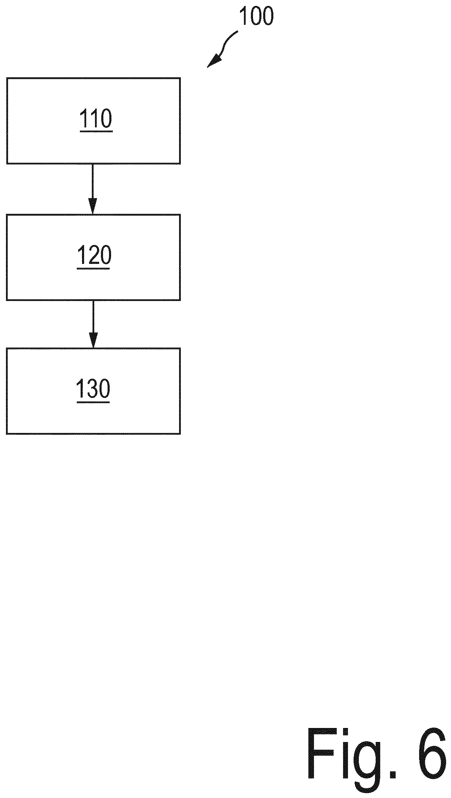

Fig. 6 is a flow-chart of a method 100 performed by the data processing system 37 (shown for the first exemplary embodiment in Fig. 1 and for the second exemplary embodiment in Fig. 5) for generating an object image (i.e. an X-ray phase-contrast image and/or an X-ray dark-field image) based on a plurality of fringe pattern images, each of which representing a Moire image (such as the Moire image 34 of Fig. 4).

The data processing system 37 reads and/or generates 110 a plurality of fringe pattern images. By way of example, the data processing system 37 may read the fringe pattern images from the image detector 7 (shown in Figs. 1 and 5) and/or from a data storage system of the data processing system 37. Additionally or alternatively, the data processing system 37 may receive signals from the image detector 7 and generates the fringe pattern images based on the received signals.

In each of the fringe pattern images, the fringe pattern has a period which is less than 20 pixels or less than 10 pixels of the respective image, wherein for each of the images a pixel pitch of the pixels of the respective image is smaller than 0.5 times or smaller than 0.3 times of the pitch of the linear fringe pattern of the respective image. A first group of the fringe pattern images are images acquired without an object being present, whereas a second group of the fringe pattern images are images acquired from an object.

The data processing system 37 then determines 120, for each pixel i of the object image, one or more analysis parameters based on the fringe pattern images, which have been acquired without an object being present. By way of example, the analysis parameters include a parameter of a spatial position of a model function, such as a parameter of a phase or a phase shift of the model function (such as the analysis parameter $\phi_i$ in the first exemplary embodiment above). The model function may describe the fringe pattern in the entrance plane of the second grating. Additionally or alternatively, the analysis parameters may include a parameter of an amplitude of the model function (such as the parameter $V_i$ in the first exemplary embodiment above) and/or a parameter of an average intensity of the model function (such as the parameter $A_i$ in the first exemplary embodiment above).

The data processing system 37 further determines 130, for each pixel i of the object image, one or more analysis parameters based on the fringe pattern images, which have been acquired from the object and further depending on the analysis parameters, which have been determined in the previous step 120 (i.e. based on the images acquired without the object). By way of example, the analysis parameters include a parameter of a phase of the model function (such as a parameter of a spatial shift of the model function generated by the object). An example for such a parameter is the analysis parameter $\psi_i$ in the first exemplary embodiment above. Additionally or alternatively, the analysis parameters may include a parameter of an amplitude of the model function (such as a parameter of a change of the amplitude of the model function generated by the object). An example for such a parameter is the parameter $D_i$ described in connection with the first exemplary embodiment above. Additionally or alternatively, the analysis parameters may include a parameter of an intensity of the model function (such as a parameter of a change of the intensity generated by the object). An example for such a parameter is the parameter $T_i$ described above in connection with the first exemplary embodiment.

One or both of the steps 120 and 130 may be performed by fitting the model function to pixel data values of the fringe pattern images. The fitting procedure may include a regression algorithm.

In view of the foregoing, an improved apparatus and an improved method are provided for generating X-ray phase-contrast images and X-ray dark-field images.

The above embodiments as described are only illustrative, and not intended to limit the technique approaches of the present invention. Although the present invention is described in detail referring to the preferable embodiments, those skilled in the art will understand that the technique approaches of the present invention can be modified or equally displaced without departing from the protective scope of the claims of the present invention. In the claims, the word "comprising" does not exclude other elements or steps, and the indefinite article "a" or "an" does not exclude a plurality. Any reference signs in the claims should not be construed as limiting the scope.

**Claims**

1. An apparatus (10; 33) for X-ray dark-field and/or X-ray phase-contrast imaging;
   the apparatus having an X-ray imaging system which comprises:

   a first and a second X-ray optical grating (1, 2) and an X-ray sensitive image detector (7) having an ordered array of X-ray sensitive pixels;

wherein the imaging system is configured to be usable with an X-ray beam (11) which traverses the first grating (1), then the second grating (2) before being incident on the X-ray sensitive detector (7) for imaging an object (4), which is positioned in the beam path of the X-ray beam (11) upstream of the first grating (1) or between the first and the second grating (1, 2);

wherein the imaging system is configured so that when no object is present, the first grating (1) generates a fringe pattern in an entrance plane of the second grating (2);

wherein the second grating (2) is configured to generate a Moire pattern from the fringe pattern so that the Moire pattern has a pitch which is less than 20 times or less than 10 times a pixel pitch of the pixels of the X-ray sensitive image detector (7);

wherein the imaging system further comprises a controller (30), which is configured to control a relative movement between the second grating (2) and the fringe pattern to acquire a Moire-image (34) at each of a plurality of relative image acquisition positions when the object is present.

2. The apparatus (10; 33) of claim 1, wherein a pixel pitch of the pixels of the detector is smaller than 0.5 times or smaller than 0.3 times the period of the Moire pattern.

3. The apparatus (10; 33) of claim 1 or 2, further comprising a data processing system (37), which is configured to: generate an X-ray dark-field image and/or an X-ray phase-contrast image based on one or more analysis parameters $(D_i, \psi_i, T_i)$; and to determine each of the one or more analysis parameters $(D_i, \psi_i, T_i)$ based on a plurality of pixel data values of one of the Moire images (34), which sample a Moire fringe pattern of the Moire image (34) over a period of the Moire fringe pattern.

4. The apparatus (10; 33) of claim 3, wherein the data processing system (37) is further configured to: determine each of the one or more analysis parameters $(D_i, \psi_i, T_i)$ further based on pixel data values of pixels of different ones of the Moire images (34).

5. The apparatus (10; 33) of any one of the preceding claims, wherein the imaging system is configured so that the Moire pattern is at least partially caused by an axis of fringes of the fringe pattern being rotated relative to an axis of the second grating (2), as seen in the entrance plane of the second grating (2); and/or a difference between a grating pitch of the second grating (2) and the pitch of the fringe pattern.

6. The apparatus (10; 33) of any one of the preceding claims, wherein the imaging system is configured so that the first and the second gratings (1, 2) form a grating interferometer; or the first grating forms a plurality of beamlets for X-ray edge illumination of the second grating.

7. The apparatus (10; 33) of any one of the preceding claims, further comprising a third grating (3), which is arranged in the beam path of the X-ray beam upstream of the first grating (1); wherein the apparatus (10; 33) is configured to at least partially generate the relative movement between the second grating (2) and the fringe pattern by controllably displacing the first, the second and/or the third grating (1, 2, 3).

8. The apparatus (10; 33) of any one of the preceding claims, wherein the imaging system is configured to determine, based on the Moire images:

   - a parameter of a spatial position of the fringe pattern in the entrance plane, measured in a direction perpendicular to a fringe axis of the fringe pattern; and/or
   - a parameter of an amplitude of the fringe pattern.

9. A method for generating an object image based on at least two fringe pattern images, each of which showing a different fringe pattern; wherein in each of the fringe pattern images, the fringe pattern has a pitch which is less than 20 pixels or less than 10 pixels of the respective image and a pixel pitch of the pixels of the respective image is smaller than 0.5 times or smaller than 0.3 times of the pitch of the fringe pattern of the respective image; wherein the method comprises:

   reading and/or generating, using a data processing system (37), the fringe pattern images and one or more position parameters for each of the fringe pattern images;

determining, using the data processing system (37), one or more analysis parameters ($D_i$, $\psi_i$, $T_i$), wherein each of the one or more analysis parameters ($D_i$, $\psi_i$, $T_i$) is determined based on the position parameters and further based on a plurality of pixel data values of a plurality of pixels of the images which comprise:

a) pixels of one of the fringe pattern images, which sample the fringe pattern of the respective image over a period of the fringe pattern; and
b) pixels of different ones of the fringe pattern images; and

wherein the method further comprises determining at least one pixel of the object image based on the one or more analysis parameters.

10. The method of claim 9, wherein:

the one or more analysis parameters ($D_i$, $\psi_i$, $T_i$) are parameters of a model function representing a linear fringe pattern; and
determining the one or more analysis parameters ($D_i$, $\psi_i$, $T_i$) comprises fitting the model function to the plurality of pixel data values using the position parameters.

11. The method of claim 10, wherein the one or more analysis parameters ($D_i$, $\psi_i$, $T_i$) comprise:

a parameter of a spatial position of the model function, and/or
a parameter of an amplitude of the model function.

12. The method of any one of claims 9 to 11, further comprising:
determining one or more analysis parameters ($D_i$, $\psi_i$, $T_i$) for each of a plurality of pixels of the object image.

13. The method of any one of claims 9 to 12, wherein each of the pixels, which are taken from different ones of the fringe pattern images, have a same or substantially a same pixel position within the respective image.

14. A program element for generating an object image based on at least two fringe pattern images, each of which showing a different fringe pattern;
wherein in each of the fringe pattern images, the fringe pattern has a pitch which is less than 20 pixels or less than 10 pixels of the respective image and a pixel pitch of the pixels of the respective image is smaller than 0.5 times or smaller than 0.3 times of the pitch of the fringe pattern of the respective image;
wherein the object image is generated using a data processing system (37), wherein the program element, when being executed by a processor of the data processing system (37), is adapted to carry out:

reading and/or generating, using the data processing system (37), the fringe pattern images and one or more position parameters for each of the fringe pattern images;
determining, using the data processing system, one or more analysis parameters ($D_i$, $\psi_i$, $T_i$), wherein each of the one or more analysis parameters ($D_i$, $\psi_i$, $T_i$) is determined based on the position parameters and further based on a plurality of pixel data values of a plurality of pixels of the images which comprise:

a) pixels of one of the images, which sample the fringe pattern of the respective image over a period of the fringe pattern; and
b) pixels of different ones of the images; and

wherein the program element, when being executed by a processor of the data processing system (37), is further adapted to carry out determining at least one pixel of the object image based on the one or more analysis parameters.

15. A computer readable medium having stored thereon the computer program element of claim 14.

Fig. 1

# Fig. 2

Fig. 3A

Fig. 3B

Fig. 3C

Fig. 4

# Fig. 5

Fig. 6

Europäisches
Patentamt

European
Patent Office

Office européen
des brevets

**EUROPEAN SEARCH REPORT**

Application Number

EP 19 17 3639

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | JP 2017 093496 A (CANON KK) 1 June 2017 (2017-06-01) * abstract * * paragraph [0001] - paragraph [0004] * * paragraph [0020] - paragraph [0039] * * figures 1-3 * | 1-15 | INV. A61B6/00 G01N23/041 |
| X | US 2016/162755 A1 (NAGAI KENTARO [JP]) 9 June 2016 (2016-06-09) * abstract * * paragraph [0030] - paragraph [0037] * * figure 1 * | 1,9,14, 15 | |
| A | | 2-8, 10-13 | |
| X | EP 2 633 814 A1 (FUJIFILM CORP [JP]) 4 September 2013 (2013-09-04) * abstract * * paragraph [0015] - paragraph [0022] * * paragraph [0050] - paragraph [0051] * * figures 1,9,10,15 * | 1,9,14, 15 | |
| A | | 2-8, 10-13 | |

TECHNICAL FIELDS
SEARCHED (IPC)

A61B
G01N

The present search report has been drawn up for all claims

| Place of search | Date of completion of the search | Examiner |
|---|---|---|
| Munich | 26 November 2019 | Montes, Pau |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

.......................................................................................................

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 19 17 3639

This annex lists the patent family members relating to the patent documents cited in the above-mentioned European search report.
The members are as contained in the European Patent Office EDP file on
The European Patent Office is in no way liable for these particulars which are merely given for the purpose of information.

26-11-2019

| Patent document cited in search report | | Publication date | Patent family member(s) | | Publication date |
|---|---|---|---|---|---|
| JP 2017093496 | A | 01-06-2017 | NONE | | |
| US 2016162755 | A1 | 09-06-2016 | JP 2016106721 | A | 20-06-2016 |
| | | | US 2016162755 | A1 | 09-06-2016 |
| EP 2633814 | A1 | 04-09-2013 | EP 2633814 | A1 | 04-09-2013 |
| | | | EP 2865336 | A1 | 29-04-2015 |
| | | | JP 5331940 | B2 | 30-10-2013 |
| | | | JP WO2012057140 | A1 | 12-05-2014 |
| | | | US 2013308750 | A1 | 21-11-2013 |
| | | | US 2015071403 | A1 | 12-03-2015 |
| | | | WO 2012057140 | A1 | 03-05-2012 |

For more details about this annex : see Official Journal of the European Patent Office, No. 12/82